# EUROPÄISCHE PATENTSCHRIFT

(11) **EP 1 058 846 B1**
(45) Veröffentlichungstag und Bekanntmachung des Hinweises auf die Patenterteilung: **14.11.2001**
(21) Anmeldenummer: 99915482.6
(22) Anmeldetag: 19.02.1999
(51) Int. Cl.: G01N 33/00

(54) **VERFAHREN UND EINRICHTUNG ZUR BESTIMMUNG VON GASFÖRMIGEN VERBINDUNGEN**
METHOD AND DEVICE FOR IDENTIFYING GASEOUS COMPOUNDS
PROCEDE ET DISPOSITIF POUR IDENTIFIER DES COMPOSES GAZEUX

(30) Priorität: 24.02.1998 DE 19807658
(43) Veröffentlichungstag der Anmeldung: 13.12.2000
(73) Patentinhaber: WMA Airsense Analysentechnik GmbH, 19061 Schwerin (DE)
(72) Erfinder: WALTE, Andreas, D-19055 Schwerin (DE); MÜNCHMEYER, Wolf, D-19055 Schwerin (DE); MATZ, Gerhard, D-21244 Buchholz (DE); HUNTE, Torsten, D-22523 Hamburg (DE); DÖHREN, Stefan, D-25499 Tangstedt (DE)
(74) Vertreter: Jaap, Reinhard
(86) Internationale Anmeldenummer: DE9900457
(87) Internationale Veröffentlichungsnummer: WO9942820

(56) Entgegenhaltungen:
- EP-A- 0 670 490
- US-A- 4 895 017
- US-A- 5 469 369
- US-A- 5 494 826
- NAKAMOTO T ET AL: "Active gas/odor sensing system using automatically controlled gas blender and numerical optimization technique" SENSORS AND ACTUATORS B, Bd. B20, Nr. 2/03, 1. Juni 1994 (1994-06-01), Seiten 131-137, XP000478152 ISSN: 0925-4005 & JP 05 273170 A (TOKYO INSTUTE OF TECHNOLOGY)

## Beschreibung

Die Erfindung betrifft ein Verfahren nach dem Oberbegriff des Anspruchs 1 und eine entsprechende Einrichtung nach dem Oberbegriff des Anspruchs 4.
Solche Verfahren und Einrichtungen werden zur Identifizierung von Gasgemischen, insbesondere in der Lebensmittelindustrie angewendet.

Es ist inzwischen von großer Bedeutung geworden, beispielsweise Lebensmittelprodukte auf ihre Qualität oder die Umwelt auf ihre Belastung zu untersuchen. Dazu werden Gasgemischproben von diesen Produkten mit angestrebten Qualitätsmerkmalen genommen und mit Hilfe geeigneter Sensoren charakteristische Abbildungen erzeugt. Diese Abbildungen werden gespeichert und gelten für alle folgenden Untersuchungen als Vergleichsmuster.

Solche Verfahren sind seit längerem bekannt. Nach diesem Verfahren arbeitende Geräte und Einrichtungen kommen in letzter Zeit verstärkt als "elektronische Nase" zum Einsatz. Diese Geräte bestehen aus einer Gasprobenahmeeinheit mit einer Pumpe und einem Gasflußsensor, einem Sensorarray mit mehreren, beispielsweise zehn unterschiedlichen Gassensoren und einem Auswerterechner. Beispielsweise Halbleitergassensoren sind unspezifische Sensoren, so daß bei Anwesenheit von Gasgemischen alle Sensoren reagieren, aber jeder Sensor ein unterschiedliches Signal liefert. Alle diese unterschiedlichen Signale ergeben ein Gesamtbilddiagramm. Die Form dieser Abbildung charakterisiert das Gasgemisch und liegt den weiteren Untersuchungen als Muster zugrunde. Es ist auch bekannt, an Stelle eines Sensorarrays mit mehreren Gassensoren ein Spektrometer, z.B. ein Ionenmobilitätsspektrometer oder ein optisches Spektroskop oder eine Kombi- nation der genannten Geräte zu verwenden.
Es hat sich aber gezeigt, daß ein derartiges Verfahren für solche Gasgemische ungeeignet ist, bei denen neben den interessierenden Verbindungen auch andere Verbindungen in viel größeren Konzentrationen auftreten. Das sind in der Regel leichtflüchtige Verbindungen, wie beispielsweise Ethanol in alkoholischen Getränken. Wegen der hohen Konzentration des Ethanols reagieren die Sensoren hauptsächlich auf das Ethanol und nicht auf die Aromastoffe und verfälschen damit die Meßergebnisse.
Ein weiterer Nachteil dieses Verfahrens besteht darin, daß viele Gassensoren bei gleichen Substanzen und unterschiedlichen Konzentrationen keine linearen Konzentrationskennlinien erzeugen. Das führt zu unterschiedlichen Mustern und damit zu einem erhöhten Aufwand zur Wiedererkennung eines Gasgemisches.
Nachteilig ist auch, daß sich die Sensoren mit der Zeit verändern und die Muster daher immer wieder erneuert werden müssen.

Aus der EP-A-0 670 490 ist nun ein Verfahren und eine Vorrichtung zum Messen eines Gasmediums mit einem chemischen Sensor bekannt geworden. Das Verfahren beruht im wesentlichen darauf, einen Probegasstrom mit einem Verdünnungsstrom in einem einstellbaren Verdünnungsverhältnis zu vermischen und das Verdünnungsverhältnis durch eine Stoffkonzentrationsmessung einer vorgegebenen Stoffkomponente zumindest nach dem Mischvorgang zu bestimmen. Ziel der Vorrichtung ist die Verdünnung des Messgases, um den nominellen Messbereich der chemischen Sensoren einzuhalten.
Dieses Verfahren und die entsprechende Vorrichtung haben Nachteile. So wird nur ein Messbereich eingehalten. Da viele Sensoren, wie z.B. Halbleitergassensoren nichtlineare Kennlinien aufweisen, werden auch in Bereichen kleinerer Meßgaskonzentrationen keine konstanten, also konzentrationsunabhängigen Muster erhalten. Weiterhin ist von Nachteil, daß das Probengas auf dem Weg zum Detektor Gaspumpen durchgueren muß. Damit ist das Verfahren und die Vorrichtung für den Nachweis von mittel- und schwerflüchtigen Verbindungen ungeeignet, weil diese Verbindungen auf diesem Wege adsorbiert und damit die Messergebnisse und die Speichereffekte verfälscht werden.
Die Vorrichtung besitzt keine Anreicherungstechnik.

Aus der T. Nakamoto et al. (1994) Sensors&Actuators B, 20, 131-137 ist ein weiteres Verfahren zur Bestimmung von gasförmigen Verbindungen beschrieben, bei dem einzelne Komponenten einem Gasgemisch solange zugemischt werden, bis eine Mischung entstanden ist, welche mit dem zu bestimmenden Gemisch identisch ist. Damit können also bekannte Gemische, wie beispielsweise Benzindämpfe guantifiziert werden. Bei diesem Verfahren wird das Meßgas nicht auf einen konstanten Wert verdünnt, so dass keine konzentrationsunabhängigen Muster oder Abbildungen erstellt werden können. Die Sensoren werden der hohen Belastung ausgesetzt, so dass geringere Lebensdauern zu erwarten sind. Mit dem vorgestellten Meßaufbau sind keine mittel- und schwerflüchtigen Gasverbin dungen nachzuweisen, da das Messgas erst über Gasflussregler geleitet wird. die wiederum die Gaszusammensetzung beeinflussen.
Auch bei diesem Verfahren ist keine Anreicherungstechnik vorgesehen.

Es besteht daher die Aufgabe, ein verfahren und eine Einrichtung der vorliegenden Gattung zu entwickeln, die die Kennlinien der einzelnen Sensoren linearisiert und konstant hält.

Eine weitere Aufgabe besteht darin, die Möglichkeit zu schaffen, die Gasproben im Bedarfsfall zusätzlich von leichtflüchtigen Gasanteilen zu befreien.

Diese Aufgabe wird verfahrensseitig durch die kennzeichnenden Merkmale des Anspruchs 1 und einrichtungsseitig durch die kennzeichnenden Merkmale des Anspruchs 4 gelöst. Zweckdienliche Ausgestaltungen ergeben sich aus den Unteransprüchen 2 und 3 und 5 bis 7.
Mit der Erfindung werden die genannten Nachteile des Standes der Technik beseitigt.
So können jetzt gasförmige Verbindungen in hoher Genauigkeit und unabhängig von ihrer Konzentration erkannt werden. Das verbessert die Qualität der Vergleichsmessungen erheblich. Dabei werden die Gassensoren dadurch geschont, daß sie nur mit einem Konzentrationsgrad weit unterhalb der Sättigungsgrenze belastet werden.
Das Verfahren ist auch insofern vorteilhaft, daß durch eine vorherige Behandlung des Probegasstromes in einer selektiven Sammeleinheit auch gasförmige Verbindungen mit selektierunfähigen Bestandteilen einer solchen Vergleichsuntersuchung unterworfen werden können. Das erweitert den Einsatzbereich des Verfahrens erheblich.
Die erfindungsgemäße Einrichtung ist durch ihr schaltbares Dreiwegeventil in der Zuleitung zum Sensorarray so ausgelegt, daß der Probegasstrom wahlweise über die selektive Sammeleinheit oder an ihr vorbei geleitet werden kann.
Diese selektive Sammeleinheit ist in besondererweise mit einer umkehrbaren Förderpumpe ausgerüstet, die durch ihre Saugleistung die Sammeleinheit füllt und durch ihre Druckleistung diese wieder entleert. Das vereinfacht die gerätetechnische Ausführung der Einrichtung.

Vorteilhaft ist auch die Verwendung allgemein bekannter Adsorbentien für die Anreicherung der selektierfähigen Gasbestandteile.

Die Erfindung soll an Hand einer schematischen Darstellung näher erläutert werden.

Die Einrichtung zur Durchführung des Verfahrens zur Bestimmung der gasförmigen Verbindung besteht in der Hauptsache aus einem Sensorenarray 1 mit beispielsweise zehn Gassensoren 2 in Form von Halbleitergassensoren. Dieses Sensorenarray 1 besitzt einerseits eine Zuleitung 3 mit einem Einlaß 4 und andererseits einen Auslaß 5. In der Zuleitung 3 befindet sich ein schaltbares Dreiwegeventil 6, an das eine selektive Sammeleinheit 7 angeschlossen ist. Diese Sammeleinheit 7 besteht aus einem speziellen Adsorber 8 und einem Heizer 9 sowie aus einer separaten Förderpumpe 10 mit einem Flußsensor 11 für die Förderpumpe 10. Zwischen dem Dreiwegeventil 6 und dem Sensorenarray 1 mündet eine Nebenleitung 12 in die Zuleitung 3 ein, in der eine Verdünnungseinheit 13 angeordnet ist. Diese Verdünnungseinheit 13 ist ebenfalls mit einer Förderpumpe 14 und einem Flußsensor 15 für die Förderpumpe 14 sowie mit einem nichtdargestellten Luftfilter ausgerüstet. Im Bereich des Auslasses 5 der Sensorenarray 1 befindet sich eine Förder- und Steuereinheit 16, die ebenfalls eine Förderpumpe 17 und eine Flußsensor 18 für die Förderpumpe 17 besitzt. Vom Sensorarray 1 zweigt eine Elektroleitung 19 ab und führt zu einem Auswerterechner 20, der über nichtdargestellte Verbindungen Kontakt zu allen Flußsensoren 11, 15 und 18 hat.

Zur Bestimmung eines Gasgemisches wird eine Probe dieses Gasgemisches genommen, in dem die Förderpumpe 17 der Förderund Steuereinheit 16 einen vorbestimmten Strom des Gasgemisches über den Einlaß 4 ansaugt und durch den Sensorarray 1 transportiert. Gleichzeitig führt die Förderpumpe 14 der Verdünnungseinheit 13 dem Probegasstrom in der Zuleitung 3 einen vorbestimmten Strom an gefilterter Frischluft zu. Dabei ist das Mischungsverhältnis von Probegasstrom und Luftstrom so gewählt, daß der Arbeitspunkt des Gasensors 2 im unteren Bereich seiner nichtlinearen Konzentrationskurve festgelegt wird. Damit reagiert der empfindlichste oder der schnellste Gassensor 2 vorzugsweise immer auf einem bestimmten Arbeitspunkt, sodaß sich unabhängig von der Konzentration des Gasgemisches immer gleiche Abbildungen von unterschiedlichen Messungen eines gleichen Probegasstromes ergeben. Über das an dem Auswerterechner 20 angezeigte und daher bekannte Mischungsverhältnis kann im Bedarfsfall und im Nachhinein die Konzentration des Gasgemisches errechnet werden.
Der Mischungsprozeß wird vom Auswerterechner 20 ständig überwacht und reguliert.
Die elektrischen Signale aller Gassensoren 2 werden am Auswerterechner 20 erfaßt, optisch abgebildet und gespeichert. Diese für den gemessenen Probegasstrom charakteristische Abbildung steht dann für die spätere Identifizierung anderer Gasgemische als Muster zur Verfügung.
Handelt es sich um ein Gasgemisch mit einem Anteil von leichtflüchtigen Bestandteilen, die wegen ihrer annähernd gleichen Konzentration nicht selektierfähig sind und die wegen ihrer hohen Konzentration hauptsächlich auf die Gassensoren 2 einwirken, dann wird die selektive Sammeleinheit 7 eingesetzt. Die Förderpumpe 10 der selektiven Sammeleinheit 7 saugt, geregelt durch den Auswerterechner 20 und den Fluß--sensor 11, den Probegasstrom in den Adsorber 8. Hier reichern sich die benötigten mittelflüchtigen und schwerflüchtigen Aromastoffe des Gasgemisches auf speziellen Adsorbentien an, während die leichtflüchtigen Gasbestandteile durchbrechen und somit nicht angereichert werden. Eine nachfolgend vom Heizer 9 eingeleitete thermische Desorption löst diese mittelflüchtigen Aromastoffe wieder von den Adsorbentien. Durch eine rechnergesteuerte Umkehrung der Förderrichtung der Förderpumpe 10 werden diese Aromastoffe wieder in die Zuleitung 3 zurückbeordert und im Zusammenwirken mit der Förderpumpe 17 der Förder- und Steuereinheit 16 in und durch den Sensorarray 1 transportiert. Gleichzeitig wird über die Verdünnungseinheit 13 in bereits beschriebener Weise wiederum gefilterte Frischluft zugemischt.

### Aufstellung der Bezugszeichen

- 1: Sensorarray
- 2: Gassensor
- 3: Zuleitung
- 4: Einlaß
- 5: Auslaß
- 6: schaltbares Dreiwegeventil
- 7: selektive Sammeleinheit
- 8: Adsorber
- 9: Heizer
- 10: Förderpumpe
- 11: Flußsensor
- 12: Nebenleitung
- 13: Verdünnungseinheit
- 14: Förderpumpe
- 15: Flußsensor
- 16: Förder- und Steuereinheit
- 17: Förderpumpe
- 18: Flußsensor
- 19: Elektroleitung
- 20: Auswerterechner

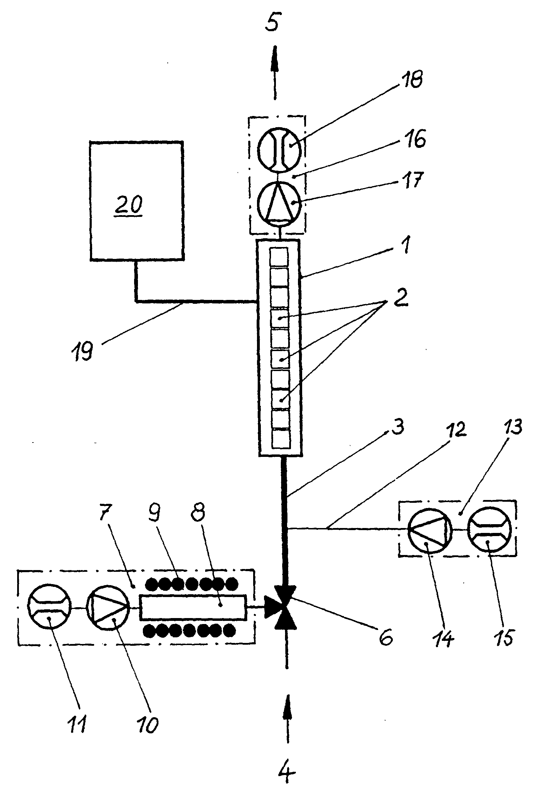

## Patentansprüche

1. Verfahren zur Bestimmung von gasförmigen Verbindungen, bei dem ein Probegasstrom durch einen Sensorarray (1) mit mehreren Gassensoren (2) geleitet wird, die elektrischen Ausgangssignale der einzelnen Gassensoren (2) von einem Auswerterechner (20) erfaßt, in ein charakteristisches Abbild ungewandelt, gespeichert und mit Abbildungen anderer Gasgemische verglichen wird,
**dadurch gekennzeichnet, daß** der Probegasstrom vor der Einleitung in den Sensorarray (1) von leichtflüchtigen Gasanteilen getrennt und befreit wird und anschließend mit einem Luftstrom verdünnt wird und dabei zur Erzielung eines vergleichbaren Abbildes die Konzentration des Probegasstromes auf einen vorbestimmten Wert unterhalb der Sättigungskonzentration konstant gehalten wird.

2. Verfahren nach Anspruch 1.
**dadurch gekennzeichnet, daß** die mittel- und schwerflüchtigen Gasanteile des Probegasstromes durch Adsorbtion von den leichtflüchtigen Gasanteilen des Probegasstromes getrennt und anschließend die angereicherten Casanteile durch thermische Desorption freigesetzt werden.

3. Einrichtung zur Bestimmung von gasförmigen Verbindungen, bestehend aus einem Sensorarray (1) mit mehreren Gassensoren (2) und einer Förder- und Steuereinheit (16) zum geregelten Transport eines Probegasstromes durch den Sensorarray (1) sowie aus einem nebengeschalteten Auswerterechner (20) zur Erfassung aller Signale des Sensorarrays (1) und zur Umwandlung dieser Signale in ein charakteristisches Abbild des Probegasstromes und zur Speicherung dieses Abbildes, **dadurch gekennzeichnet, daß** in eine Zuleitung (4) des Sensorarrays (1) ein schaltbares Dreiwegeventil (6) angeordnet ist, das mit einer selektiven Sammeleinheit (7) zur Trennung und Befreiung von leichtflüchtigen Gasanteilen Verbindung hat und zwischen dem Dreiwegeventil (6) und dem Sensorarrays (1) eine Nebenleitung (12) mit einer Verdünnungseinheit (13) zur Konstanthaltung der Konzentration des Probegasstromes auf einen vorbestimmten Wert unterhalb der Sättigungskonzentration einmündet.

4. Einrichtung nach Anspruch 3,
**dadurch gekennzeichnet, daß** die selektive Sammeleinheit (7) mit einer regelbaren und in der Förderrichtung umkehrbaren Förderpumpe (10) ausgerüstet ist.

5. Einrichtung nach Anspruch 4,
**dadurch gekennzeichnet, daß** die selektive Sammeleinheit (7) aus einem Adsorber (8) und einem Heiser (9) sowie aus einer separaten Förderpumpe (10) mit einem Flußsensor (11) für die Förderpumpe (10) besteht.

6. Einrichtung nach Anspruch 3,
**dadurch gekennzeichnet, daß** die Verdünnungseinheit (13) aus einer regelbaren Förderpumpe (14), einem Flußsensor (15) für die Förderpumpe (14) und einem Filter besteh.

## Claims

1. A process for determining gaseous compounds, wherein a stream of test gas is passed through a sensor array (1) comprising several gas sensors (2), the electrical output signals of the individual gas sensors (2) are registered by an evaluating computer (20), converted into a characteristic image, stored and compared with images of other gas mixtures,
**characterised in that** prior to being introduced into the sensor array (1) the stream of test gas is separated from readily volatile gas portions and liberated therefrom and is subsequently diluted with a stream of air and in the process, with a view to achieving a comparable image, the concentration of the stream of test gas is kept constant at a predetermined value below the saturation concentration.

2. Process according to Claim 1,
**characterised in that** the medium-volatility and low-volatility gas portions of the stream of test gas are separated from the readily volatile gas portions of the stream of test gas by adsorption and subsequently the enriched gas portions are liberated by thermal desorption.

3. A device for determining gaseous compounds,
consisting of a sensor array (1) comprising several gas sensors (2) and of a conveying and controlling unit (16) for the regulated transport of a stream of test gas through the sensor array (1) and also consisting of an evaluating computer (20) connected in parallel for registering all the signals of the sensor array (1) and for converting these signals into a characteristic image of the stream of test gas and for storing this image, **characterised in that** a switchable three-way valve (6) which has a connection to a selective collecting unit (7) for separating and liberating readily volatile gas portions is arranged in a supply line (3) of the sensor array (1), and between the three-way valve (6) and the sensor array (1) there leads in a branch line (12) with a dilution unit (13) for keeping the concentration of the stream of test gas constant at a predetermined value below the saturation concentration.

4. Device according to Claim 3,
**characterised in that** the selective collecting unit (7) is equipped with a controllable delivery pump (10) that is capable of having its delivery direction reversed.

5. Device according to Claim 4,
**characterised in that** the selective collecting unit (7) consists of an adsorber (8) and a heater (9) and also of a separate delivery pump (10) with a flow sensor (11) for the delivery pump (10).

6. Device according to Claim 3,
**characterised in that** the dilution unit (13) consists of a controllable delivery pump (14), a flow sensor (15) for the delivery pump (14) and a filter.

## Revendications

1. Procédé de détermination de composés gazeux, selon lequel on fait passer un courant de gaz échantillon à travers un système de capteurs (1) à plusieurs capteurs de gaz (2), un calculateur d'exploitation (20) saisit les signaux de sortie électriques des différents capteurs de gaz (2) les convertit en un diagramme caractéristique, le met en mémoire et le compare aux diagrammes d'autres mélanges gazeux,
**caractérisé en ce que**
le courant de gaz échantillon est séparé et libéré des particules gazeuses légères et volatiles, puis dilué par un courant d'air, et la concentration de ce courant gazeux est alors maintenue constante, en vue d'obtenir un diagramme comparable, à une valeur prédéterminée, au dessous de la concentration de saturation.

2. Procédé suivant la revendication 1,
**caractérisé en ce que**
les particules gazeuses moyennement et peu volatiles du courant gazeux sont séparées des particules très volatiles de ce courant gazeux, par adsorption, puis les particules ainsi enrichies sont libérées par désorption thermique.

3. Installation de détermination de composés gazeux, ayant un système de capteurs (1) avec plusieurs capteurs de gaz (2) et une unité d'alimentation et de commande (16) pour assurer le débit régulé d'un courant gazeux échantillon à travers le système de capteurs (1), connecté à un calculateur d'exploitation (20) recueillant l'ensemble des signaux du système de capteurs (1), transformant ces signaux en un diagramme caractéristique de ce courant gazeux échantillon et mettant en mémoire ce diagramme,
**caractérisé en ce que**
dans une alimentation (4) au système de capteurs (1) est installée une vanne 3-voies commutable (6) reliée à une unité collectrice (7) sélective destinée à séparer et libérer les particules gazeuses très volatiles et, entre la vanne-3 voies (6) et le système de capteurs (1), débouche une dérivation (12) munie d'une unité de dilution (13), pour maintenir constante la concentration du courant gazeux à une valeur prédéterminée, au dessous de la concentration de saturation.

4. Installation selon la revendication 3,
**caractérisée en ce que**
l'unité collectrice (7) sélective est équipée d'une pompe d'alimentation (10) réglable, et réversible dans le sens d'alimentation.

5. Installation suivant la revendication 4,
**caractérisée en ce que**
l'unité collectrice (7) sélective se compose d'un adsorbeur (8) et d'un élément chauffant (9) ainsi que d'une pompe d'alimentation (10) séparée, avec un capteur de débit (11) pour cette pompe (10).

6. Installation selon la revendication 3,
**caractérisée en ce que**
l'unité de dilution (13) se compose d'un pompe d'alimentation réglable (14), d'un capteur de débit (15) pour la pompe (14) et d'un filtre.
